Europäisches Patentamt

**European Patent Office**

Office européen des brevets

(11) Numéro de publication: **0 301 951 B1**

(12) **FASCICULE DE BREVET EUROPEEN**

(45) Date de publication du fascicule du brevet:
**14.11.90**

(51) Int. Cl.⁵: **C03B 5/027**, C03B 5/26, C03B 3/00

(21) Numéro de dépôt: **88401928.2**

(22) Date de dépôt: **25.07.88**

(54) Procédé et dispositif pour le traitement de laine de verre usagée en vue de sa mise au rebut.

(30) Priorité: **30.07.87 FR 8710808**

(43) Date de publication de la demande:
**01.02.89 Bulletin 89/5**

(45) · Mention de la délivrance du brevet:
**14.11.90 Bulletin 90/46**

(84) Etats contractants désignés:
**AT BE CH DE ES FR GB GR IT LI LU NL SE**

(56) Documents cités:
**EP-A- 0 041 635**
**DE-A- 2 003 544**
**DE-A- 2 420 648**
**DE-B- 2 842 505**
**FR-A- 2 593 092**
**GB-A- 2 116 538**
**US-A- 3 429 972**
**US-A- 4 213 002**

(73) Titulaire: **WANNER ISOFI, 250 Route de l'Empereur, F-92508 Rueil-Malmaison(FR)**

(72) Inventeur: **Maugendre, Stéphane, 13 Allée des Bourgognes, F-60500 Chantilly(FR)**

(74) Mandataire: **Luziau, Nelly et al, Saint Gobain Recherche 39, Quai Lucien Lefranc, F-93304 Aubervilliers Cédex(FR)**

ACTORUM AG

## Description

La présente invention concerne le traitement de laine de verre usagée en vue de sa mise au rebut. Elle concerne plus particulièrement le traitement de laine de verre en provenance d'usines nucléaires.

On sait que divers dispositifs des centrales nucléaires sont isolés thermiquement par de la laine de verre, qui, à l'issue d'une période plus ou moins longue, peut être éventuellement contaminée par des matières radioactives.

Lorsque des interventions ont lieu sur ces dispositifs, en vue de leur maintenance ou de réparations éventuelles, la laine de verre contaminée doit être récupérée sur le site, traitée et conditionnée dans des fûts étanches.

Du fait de son volume apparent, la laine de verre pose toutefois de sérieux problèmes de manutention et de stockage. En effet, c'est par dizaines de mètres cubes que cette laine de verre est utilisée comme isolant thermique et un générateur de vapeur standard d'une centrale nucléaire comprendra par exemple 80 m³ de cet isolant thermique.

On peut, bien entendu, réduire considérablement ce volume, par exemple d'un facteur de 10, en procédant à un broyage de la laine, mais les volumes à récupérer demeurent toujours importants, et la forme du matériau récupéré (fibres broyées de petites dimensions) se prête mal à une menutention et à une récupération en conteneurs stériles.

Pour traiter la laine de verre usagée, particulièrement la laine de verre contaminée par des matières radioactives, on a proposé comme le décrit la demande de brevet français FR-A 2 593 092, un procédé qui consiste à broyer la laine de verre pour obtenir des flocons d'une dimension de l'ordre du centimètre, à les fondre dans un four électrique comprenant une batterie d'éléments chauffants électriques disposés sous la voûte du four.

La laine de verre ainsi traitée est récupérée sous forme de verre fondu dans des conteneurs appropriés. Le four électrique utilisé fait partie d'un dispositif autonome, d'encombrement limité, qui peut être transporté sur le site même où la laine de verre doit être traitée.

Bien que le procédé et le dispositif décrits à la demande de brevet français FR-A 2 593 092 permettent de réduire efficacement le volume de la laine de verre usagée à rebuter, ils présentent cependant certains inconvénients. En effet, avant d'être fondue, la laine de verre doit être broyée. Elle se trouve donc sous une forme non compacte qui rend difficile son introduction dans le four.

D'autre part, le broyage constitue une étape qui est non seulement supplémentaire dans le processus de traitement de la laine de verre pour sa mise rebut, mais en outre génante lorsque la laine de verre est contaminée par des matières radioactives ; en effet, le broyage de la laine de verre contaminée nécessite, dans les centrales nucléaires, des installations spécifiques pour l'élimination de la poussière formée lors du broyage, ce qui augmente le coût de cette opération de mise au rebut.

Un autre inconvénient du procédé décrit à la demande de brevet précitée résulte du fait que les moyens de chauffage sont situés sous la voûte du four. La laine de verre broyée introduite dans le four forme une barrière isolante entre les éléments chauffants de la voûte et la mass fondue, ce qui empêche une fusion rapide de la laine de verre. Pour éviter cet inconvénient, la laine de verre doit être intro duite par petites quantités successives, ce qui augmente la durée d'utilisation du four et en diminue le rendement.

La présente invention a pour objet un procédé de traitement de la laine de verre usagée, en vue de sa mise au rebut, qui évite les inconvénients précités, en particulier qui ne nécessite pas une étape de broyage, et dont le rendement est amélioré.

La présente invention a aussi pour objet un dispositif permettant la mise en oeuvre du procédé de traitement de la laine de verre usagée, qui puisse éventuellement être transporté sur le site même où la laine doit être traitée.

Le procédé, selon l'invention, pour le traitement de la laine de verre usagée en vue de sa mise au rebut sous un faible volume, suivant lequel on fond la laine de verre dans un four électrique et on la récupère pour la rebuter, est caractérisé en ce que l'on introduit des blocs de laine de verre usagée au-dessus de la surface libre d'une mas se de verre fondu contenue dans un four à électrodes, on presse les blocs vers la surface libre du verre fondu pour assurer un contact intime entre le verre fondu et la laine de verre et on récupère la masse fondue obtenue dans un conteneur.

Le dispositif selon l'invention pour le traitement de la laine de verre usagée, en vue de sa mise au rebut, comprend un four électrique à électrodes apte à contenir une masse de verre fondu et dont la sole est équipée d'un moyen d'évacuation vers l'extérieur de la masse fondue, un moyen d'alimentation en laine de verre de ce four et un organe presseur pour exercer une pression sur des blocs de laine de verre introduits dans le four, dirigée vers la surface libre de la masse de verre fondu et suffisante pour assurer une mise en contact intime entre la masse de verre fondu et les blocs de laine de verre.

Le procédé selon l'invention permet de récupérer sous une forme fondue et donc moins volumineuse, des matériaux d'isolation tels que la laine de verre ou la laine de roche. Pour plus de commodité, on ne fera référence, dans la présente description, qu'à la laine de verre, étant bien entendu que la laine de roche peut aussi être traitée par le procédé selon l'invention.

Le procédé selon l'invention peut traiter de la laine de verre quelle que soit la forme sous laquelle elle se présente. On peut donc l'introduire dans le four sous sa forme brute d'utilisation, par exemple sous forme de panneaux, sans qu'il soit nécessaire de lui faire subir un traitement préalable particulier, tel qu'un broyage. Lorsque la laine de verre usagée est sous forme de flocons, elle est habituellement, après récupération des installations où elle est utilisée, mise en sacs, notamment pour faciliter sa manipulation. Ces sacs, qu'ils contiennent des flocons ou bien éventuellement des panneaux, peuvent être introduits directement dans le four. Les termes "blocs de laine de verre" sont utilisés dans la présente description pour faire référence à la masse compacte que présente des panneaux ou des sacs contenant de la laine de verre.

Au dessin annexé, donné seulement à titre d'exemple, la figure unique représente schématiquement un dispositif pour la mise en oeuvre du procédé de traitement de laine de verre usagée suivant l'invention.

Sur cette figure, il apparait que ce dispositif comprend un four électrique (1) présentant une paroi périphérique (2) et une sole (3), aptes à contenir une masse de verre fondu (4). Des électrodes (5), servant à la fusion du verre, sont montées sur des supports (6) mobiles et refroidies, par des moyens usuels non représentés, dans la zone de transition entre le verre et l'atmosphère. Le four (1) est équipé d'une hotte étanche (7) de protection reliée à un conduit (8) raccordé à un système classique de dépoussiérage et d'évacuation de fumées, non représenté.

Suivant l'invention, un organe presseur (9) est placé dans la hotte (7). Il est constitué d'une plaque sensiblement parallèle à la surface libre de la masse de verre fondu (4) et reliée à un vérin (10) servant à la déplacer, à l'intérieur de la hotte (7), à partir d'une position inactive (9'), vers la surface libre de la masse de verre fondu portant des blocs de laine de verre (11) pour exercer sur ceux-ci, en une position telle que (9"), une pression suffisante pour assurer une mise en contact intime entre la masse de verre fondu et les blocs de laine de verre. La sole (3) du four est munie d'un moyen d'évacuation (12) de la masse fondue vers un conteneur non représenté.

Le dispositif de traitement de la laine de verre est monté sur un chassis (13) en vue de son transport sur le site de traitement.

On va décrire maintenant la mise en oeuvre de procédé de traitement selon l'invention à l'aide du dispositif décrit précédemment.

Le procédé selon l'invention consiste à introduire de la laine de verre (11) pour la fondre au-dessus de la surface libre d'une masse de verre fondu (4) formée dans un four électrique (1) à électrodes (5), puis à lui appliquer une pression pour la mettre en contact intime avec la masse de verre fondu, à l'aide de l'organe presseur (9).

Pour obtenir cette masse de verre fondu (4), on place dans le four (1) du calcin soso-calcique finement broyé qui est, de préférence, réduit, c'est-à-dire sans sulfate, pour éviter la formation de mousse. On introduit les électrodes (5) dans la calcin en déplaçant les supports (6) pour qu'une hauteur suffisante d'électrodes soit immergée dans le calcin. La profondeur d'immersion des électrodes tient compte, comme il est usuel, de l'épaisseur de calcin introduit dans le four et peut varier pour assurer une homogénéisation thermique souhaitée dans toute la masse de verre fondu. En utilisant tout moyen approprié, par exemple un brûleur, on fait fondre en surface le calcin entre les électrodes. Celles-ci sont alors mises sous tension. L'accrochage électrique a lieu dès que le verre est suffisamment chaud entre les deux électrodes. Par rayonnement thermique, le calcin fond de proche en proche. On peut alors ajouter progressivement du calcin pour compléter le niveau du bain de verre fondu. Le procédé de formation de bain de verre fondu dans un four électrique à partir de calcin est bien connu de l'homme de métier et ne nécessite donc pas une description détaillée.

Lorsque l'on a obtenu un bain de verre fondu approprié, on introduit des blocs de laine de verre (11) par tout moyen d'alimentation approprié. Comme on l'a indiqué précédemment, la laine de verre peut se présenter sous sa forme brute d'utilisation telle que des panneaux (11). Lorsqu'elle se trouve dans des sacs, ceux-ci sont de préférence en papier ou en polyéthylène, matières qui, transformées en fumées lors de leur combustion, n'ont pas d'action nuisible sur la fusion du verre. On introduit ces blocs de laine de verre sur la surface libre de la masse de verre fondu et de préférence de façon à ce qu'ils ne la recouvrent pas totalement pour permettre le dégazage du verre.

On actionne alors le vérin (10) pour faire descendre l'organe presseur (9) à partir d'une position inactive (9') vers la surface libre du bain de verre fondu (4) portant les blocs de laine de verre (11) pour exercer sur ceux-ci une pression telle que les blocs de laine s'enfoncent dans le bain de verre fondu, assurant ainsi un contact intime entre le verre fondu et la laine de verre et par suite, une fusion rapide de cette dernière. Avant que la totalité de la laine de verre ne soit fondue, on relève l'organe presseur pour introduire une nouvelle charge de laine de verre et le cycle continue.

Le niveau de verre fondu dans la four est maintenu constant en soutirant en continu le verre fondu et on récupère la masse fondue dans un conteneur, non représenté sur la figure, situé sous le moyen d'évacuation (12) de la sole (3). Le débit de verre évacué est contrôlé en faisant varier sa viscosité.

Pendant toute la mise en oeuvre du procédé selon l'invention, la hotte (7) est maintenue en légère dépression par rapport à l'atmosphère extérieure, par des moyens d'aspiration classiques pour éliminer les fumées résultant de la combustion des sacs contenant la laine de verre et des matières organiques présentes dans la laine de verre (par exemple la colle), par le conduit d'évacuation (8) équipé d'éléments filtrants, non représentés sur la figure.

Le procédé selon l'invention, que l'on vient de décrire, présente un avantage important par rapport à celui décrit à la demande de brevet FR-A 2 503 092, mentionnée précédemment. En effet, comme on l'a indiqué, ce procédé permet de traiter de la laine de verre usagée quelle que soit sa forme et ne nécessite pas une étape de broyage préalable à l'introduction dans le four, qui présente les inconvénients précités. Ainsi, le procédé permet d'éviter une installation de dispositifs de broyage sur les sites de récupération de la substance isolante usagée, installation qui est particulièrement coûteuse lorsque ces sites sont des usines nucléaires.

Le procédé selon l'invention permet aussi d'éviter une manipulation difficile de la laine de verre sous forme de flocons et son introduction dans les fours. Ainsi, au gain de temps associé au fait qu'une étape de traitement est supprimée, s'ajoute l'avantage d'une manipulation plus aisée, plus rapide (puisqu'il n'est pas nécessaire de mettre la laine de verre en sacs lorsqu'elle se présente sous forme de panneaux) et celui d'un coût de fonctionnement moins élevé. On doit aussi noter que les moyens de chauffage du four ne se trouvant pas sous la voûte, la laine de verre ne forme pas une barrière isolante entre ce éléments et la masse de verre fondu et une quantité plus importante de laine de verre peut être introduite dans le four sans diminuer la vitesse de fusion et le four peut fonctionner en continu.

Comme on l'a indiqué précédemment, il est important, lorsque la laine de verre à traiter provient d'usines nucléaires, de pouvoir la traiter sur place dans des dispositifs essentiellement transportables fonctionnant par campagnes de courte durée.

La paroi périphérique (12) et la sole (3) du four peuvent être constituées par tout matériau approprié pour les fours de fusion du verre. Il est peut être souhaitable d'utiliser un matériau autre que la matière réfractaire habituellement utilisée pour les fours, de manière à en diminuer le poids et à les rendre moins fragiles aux chocs et permettre ainsi un transport plus facile de l'ensemble du dispositif de traitement de la laine de verre.

D'autre part, ce dispositif devant servir pour des campagnes de courte durée, il est souhaitable d'éviter les étapes d'attrempage et de désattrempage, nécessaires lors de chaque mise en fonctionnement et arrêt des fours en matériau réfractaire, étapes qui augmentent la durée des campagnes et provoquent une usure plus rapide des fours. Aussi le four utilisé de préférence dans l'invention, comprend une paroi périphérique constituée d'une double enveloppe métallique (2') dans laquelle circule de l'eau pour son refroidissement. Tout métal résistant à la corrosion due au verre fondu à haute température peut être utilisé, l'acier ordinaire donnant des résultats satisfaisants.

La sole (3) du four peut être formée par exemple de dalles en matériau réfractaire (3') pour isoler le four contre les pertes thermiques ; ces dalles sont recouvertes de béton réfractaire (3") pour éviter les fuites de verre fondu par les interstices entre les dalles réfractaires. La sole est posée sur un chassis métallique refroidi.

La sole (3) peut aussi être formée, comme la paroi périphérique, d'une double enveloppe métallique refroidie par de l'eau et recouverte de béton réfractaire.

Le four est avantageusement basculant autour de pivots non représentés de manière à pouvoir le vider plus rapidement si cela est nécessaire, notamment pour des raisons de sécurité.

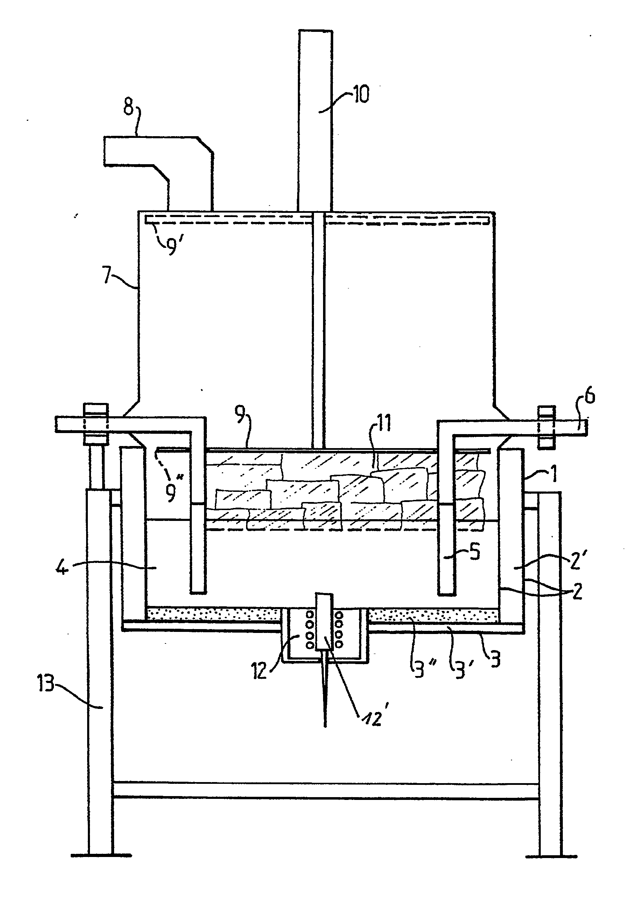

Les électrodes (5) sont par exemple des électrodes en molybdène habituellement utilisées pour la fusion électrique du verre. La figure (1) ne représente que deux électrodes ; mais il est bien évident qu'un nombre supérieur d'électrodes peut être utilisé en fonction de la dimension du four. Les électrodes peuvent plonger dans le bain de verre fondu, comme le montre la figure 1. Elles sont montées classiquement sur des supports (6) qui sont de préférence mobiles, permettant ainsi de faire varier la profondeur d'immersion des électrodes dans le bain de verre fondu. Les électrodes sont refroidies par des moyens usuels, non représentés afin d'éviter leur oxydation dans la zone de transition entre le verre et l'atmosphère. On peut régler la distance de pénétration des électrodes dans le verre et leur écartement par tout moyen approprié, par exemple par des vérins hydrauliques. On peut utiliser avantageusement par exemple trois électrodes en molybdène, alimentées en courant triphasé.

Les fours électriques utilisables dans l'invention sont bien connus de l'homme du métier et il n'est pas nécessaire de donner une description plus détaillée de ces fours et de leur fonctionnement.

L'organe presseur (9) peut avoir toute forme appropriée au four utilisé et il est constitué en toute matière ayant une rigidité suffisante pour appliquer la pression souhaitée sur les blocs de laine de verre pour leur assurer un contact intime avec la masse de verre fondu. L'organe presseur peut être par exemple en acier ordinaire.

De préférence, l'organe presseur est constitué par une plaque munie de découpes pour le passage des électrodes.

La sole (3) du four est équipée d'un moyen d'évacuation (12) du verre fondu vers l'extérieur. Tout moyen approprié peut être utilisé. Ce peut être une buse (12') en molybdène chauffée par induction ou bien en platine chauffée par effet Joule. Le diamètre de sortie de la buse peut varier et est déterminée suivant le débit de verre fondu souhaité.

Pour introduire la laine de verre dans le four, divers types de convoyeurs peuvent être utilisés.

Le raccordement du convoyeur au four est réalisé à l'aide d'un sas permettant de maintenir le four en dépression pour les raisons indiquées plus haut.

**Revendications**

1. Procédé de traitement de laine de verre usagée, en vue de sa mise au rebut, consistant à fondre la laine de verre dans un four électrique et à récupérer le matériau fondu pour le rebuter, caractérisé en ce que l'on introduit des blocs de laine de verre (11) usagée au dessus de la surface libre d'une masse de verre fondu (4) contenue dans un four à électrodes (1), on presse les blocs vers la surface libre du verre fondu pour assurer une mise en contact intime entre le verre fondu et la laine et on récupère la masse fondue dans un conteneur.

2. Procédé conforme à la revendication 1, caractérisé en ce que, avant l'introduction des blocs de laine de verre (11), on fait fondre une quantité suffisante de calcin réduit finement broyé.

3. Procédé conforme à l'une quelconque des revendications 1 et 2, caractérisé en ce qu'on introduit les blocs de laine de verre (11) dans le four (1) de manière qu'une partie de la surface libre du bain de verre ne soit pas recouverte par ces blocs.

4. Procédé conforme à l'une quelconque des revendications 1 à 3, caractérisé en ce qu'on introduit la laine de verre (11) sous forme de panneaux, éventuellement dans un sac en polyéthylène ou en papier.

5. Procédé conforme à l'une quelconque des revendications 1 à 3, caractérisé en ce qu'on introduit la laine de verre (11) sous forme broyée dans un sac en papier ou en polyéthylène.

6. Dispositif pour le traitement de laine de verre (11) usagée en vue de sa mise au rebut, comprenant un four électrique (1), apte à contenir une masse de verre fondue (4) et dont la sole (3) est équipée d'un moyen d'évacuation (12), vers l'extérieur, du verre fondu et un moyen d'alimentation en laine de verre de ce four, caractérisé en ce que le four électrique (1) est un four à électrodes et en ce qu'il comprend en outre un organe presseur (9) pour exercer une pression sur les blocs de laine de verre introduits dans le four, dirigée vers la surface libre de la masse de verre fondu et suffisante pour assurer une mise en contact intime entre la masse de verre fondu contenu dans le four et les blocs de laine de verre.

7. Dispositif selon la revendication 6, caractérisé en ce que le four (1) comprend une paroi périphérique (2) constituée d'une double enveloppe métallique (2') dans laquelle circule de l'eau pour son refroidissement.

8. Dispositif selon la revendication 7, caractérisé en ce que le four comprend une sole en matériau réfractaire (3') recouvert de béton réfractaire (3"), posée sur un chassis métallique refroidi (13).

9. Dispositif selon la revendication 7, caractérisé en ce que le four (1) comprend une sole formée d'une double enveloppe métallique refroidie par circulation d'eau, recouverte de béton réfractaire (3").

10. Dispositif selon l'une quelconque des revendications 6 à 9, caractérisé en ce que le moyen d'évacuation (12) du verre fondu (4) est constitué par une buse (12') en molybdène chauffée par induction.

11. Dispositif selon l'une quelconque des revendications 6 à 9, caractérisé en ce que le moyen d'évacuation (12) du verre est constitué par une buse (12') en platine chauffée par effet Joule.

12. Dispositif selon l'une quelconque des revendications 6 à 11, caractérisé en ce que le four (1) comprend trois électrodes (5) plongeantes en molybdène.

13. Dispositif selon l'une quelconque des revendications 6 à 12, caractérisé en ce que l'organe presseur est constitué par une plaque actionnée par un vérin (10) et munie de découpes pour le passage des électrodes (5).

14. Dispositif conforme à l'une quelconque des revendications 6 à 13 caractérisé en ce qu'il comprend des moyens d'aspiration et des moyens filtrants pour mettre le four en dépression, évacuer et filtrer les fumées.

15. Dispositif conforme à l'une quelconque des revendications 6 à 14, caractérisé en ce qu'il comprend un moyen pour introduire la laine de verre (11) dans le four (1) par un sas assurant le maintien en dépression du four.

**Patentansprüche**

Verfahren zur Behandlung von Altglaswolle zu deren Entsorgung, welches das Schmelzen der Glaswolle in einem elektrischen Ofen und Auffangen des geschmolzenen Altmateriales umfaßt, dadurch gekennzeichnet,
– daß man über der freien Oberfläche einer geschmolzenen Glasmasse (4), die in einem Elektrodenofen (1) enthalten ist, Blöcke (11) aus Altglaswolle einführt;
– daß man die Blöcke gegen die freie Oberfläche des geschmolzenen Glases preßt, um ein enges In-Kontakt-Bringen des geschmolzenen Glases und der Wolle zu gewährleisten; und
– daß man die geschmolzene Masse in einem Behälter sammelt.

2. Verfahren nach Anspruch 1, dadurch gekennzeichnet, daß man vor der Einführung der Blöcke (11) aus Glaswolle eine genügende Menge an reduziertem fein gemahlenem Glasbruch schmilzt.

3. Verfahren nach einem der Ansprüche 1 und 2, dadurch gekennzeichnet, daß man die Blöcke (11) aus Glaswolle in den Ofen (1) so einführt, daß ein Teil der freien Oberfläche des Glaßbades nicht durch diese Blöcke bedeckt wird.

4. Verfahren nach einem der Ansprüche 1 bis 3, dadurch gekennzeichnet, daß man die Glaswolle (11) in Plattenform, gegebenenfalls in einem Sack aus Polyethylen oder aus Papier, einführt.

5. Verfahren nach einem der Ansprüche 1 bis 3, dadurch gekennzeichnet, daß man die Glaswolle (11) in gemahlener Form in einem Sack aus Papier oder aus Polyethylen einführt.

6. Vorrichtung zur Behandlung von Altglaswolle (11) zur Entsorgung, mit einem elektrischen Ofen (1), zur Aufnahme einer Masse aus geschmolzenem Glas (4), dessen Sohle (3) mit einer Austrageinrichtung (12), für geschmolzenes Glas nach außen und einer Beschickungseinrichtung des Ofens für Glaswolle versehen ist, dadurch gekennzeichnet,
– daß der elektrische Ofen (1) ein Elektrodenofen ist; und
– daß er darüberhinaus ein Preßelement (9) zum Ausüben eines Druckes auf die Mineralwolleblöcke umfaßt, welche in den Ofen eingeführt werden, gegen die freie Oberfläche der Masse des geschmolzenen Glases gerichtet und ausreichend, um einen engen Kontakt zwischen der Masse des geschmolzenen Glases, welche in dem Ofen enthalten ist, und den Blöcken aus Glaswolle zu gewährleisten.

7. Vorrichtung nach Anspruch 6, dadurch gekennzeichnet, daß der Ofen (1) eine Außenwand (2) aufweist, die aus einem metallischen Doppelmantel (2') aufgebaut ist, worin Wasser zu ihrer Kühlung zirkuliert.

8. Vorrichtung nach Anspruch 7, dadurch gekennzeichnet, daß der Ofen (1) eine Sohle aus feuerfestem Material (3') aufweist, welche mit feuerfestem Beton (3") bedeckt ist, wobei sie auf einem gekühlten Metallchassis (13) angeordnet ist.

9. Vorrichtung nach Anspruch 7, dadurch gekennzeichnet, daß der Ofen (1) eine Sohle umfaßt, die aus einem metallischen Doppelmantel gebildet

ist, die durch Wasserzirkulation gekühlt wird und die mit feuerfestem Beton (3") bedeckt ist.

10. Vorrichtung nach einem der Ansprüche 6 bis 9, dadurch gekennzeichnet, daß die Austrageinrichtung (12) für das geschmolzene Glas (4) durch eine induktionsbeheizte Molybdändüse (12') gebildet ist.

11. Vorrichtung nach einem der Ansprüche 6 bis 9, dadurch gekennzeichnet, daß die Austrageinrichtung (12) für das Glas aus einer mit einer elektrischen Widerstandsheizung versehenen Platindüse (12') gebildet ist.

12. Vorrichtung nach einem der Ansprüche 6 bis 11, dadurch gekennzeichnet, daß der Ofen (1) drei Eintauchelektroden (5) aus Molybdän umfaßt.

13. Vorrichtung nach einem der Ansprüche 6 bis 12, dadurch gekennzeichnet, daß das Preßelement durch eine Platte gebildet ist, die über eine Stellvorrichtung (10) betrieben wird und mit Ausnehmungen für den Durchtritt der Elektroden (5) versehen ist.

14. Vorrichtung nach einem der Ansprüche 6 bis 13, dadurch gekennzeichnet, daß sie Ansaugeinrichtungen und Filtereinrichtungen umfaßt, um den Ofen unter Unterdruck zu setzen, wobei die Dämpfe abgesaugt und filtriert werden.

15. Vorrichtung nach einem der Ansprüche 6 bis 14, dadurch gekennzeichnet, daß sie eine Einrichtung zum Einführen der Glaswolle (11) in den Ofen (1) durch eine Schleuse umfaßt, um zu gewährleisten, daß der Ofen unter Unterdruck bleibt.

**Claims**

1. A method of processing used glass wool so that it may be scrapped, consisting of melting the glass wool in an electric furnace and recovering the molten material in order to scrap it, characterised in that blocks (11) of used glass wool are placed on the exposed surface of a mass (4) of molten glass contained in a furnace (1) comprising electrodes, the blocks are pressed down onto the exposed surface of the molten glass in order to ensure intimate contact between the molten glass and the wool and in that the molten mass is recovered in a container.

2. A method according to claim 1, characterised in that prior to introducing blocks (11) of glass wool, a sufficient quantity of crushed and finely comminuted waste glass is melted.

3. A method according to either of claims 1 or 2, characterised in that blocks (11) of glass wool are introduced into the furnace (1) so that a part of the exposed surface of the glass bath is not covered by these blocks.

4. A method according to any one of claims 1 to 3, characterised in that the glass wool (11) is introduced in the form of panels, possibly in a polyethylene or paper bag.

5. A method according to any one of claims 1 to 3, characterised in that the glass wool (11) is introduced in crushed form in a paper or polyethylene bag.

6. An apparatus for processing used glass wool (11) with a view to its being scrapped, and comprising an electric furnace (1) adapted to contain a mass (4) of molten glass and of which the hearth (3) is equipped with means (12) for discharging molten glass to the outside of the furnace and with means of supplying glass wool to the furnace, characterised in that the electric furnace (1) is a furnace with electrodes and in that it also comprises pressing means (11) for exerting pressure on the blocks of glass wool introduced into the furnace, directed towards the exposed surface of the mass of molten glass and sufficient to ensure intimate contact between the blocks of glass wool and the mass of molten glass which is contained in the furnace.

7. An apparatus according to claim 6, characterised in that the furnace (1) comprises a peripheral wall (2) consisting of a double metal jacket (2') in which cooling water circulates.

8. An apparatus according to claim 7, characterised in that the furnace comprises a hearth (3') of refractory material covered with refractory concrete (3") placed on a cooled metal frame (13).

9. An apparatus according to claim 7, characterised in that the furnace (1) comprises a hearth consisting of a double metal casing cooled by circulating water and covered with refractory concrete (3").

10. An apparatus according to any one of claims 6 to 9, characterised in that the means (12) of discharging molten glass (4) consists of an induction heated molybdenum nozzle (12').

11. An apparatus according to any one of claims 6 to 9, characterised in that the glass discharging means (12) consist of a platinum nozzle (12') heated by Joules' effect.

12. An apparatus according to any one of claims 6 to 11, characterised in that the furnace (1) comprises three molybdenum immersion electrodes (5).

13. An apparatus according to any one of claims 6 to 12, characterised in that the pressing means consist of a plate operated by a jack (10) and provided with cut-outs through which the electrodes (5) can pass.

14. An apparatus according to any one of claims 6 to 13, characterised in that it comprises vacuum extraction means and filtering means to place the furnace under negative pressure and to extract and filter the fumes.

15. An apparatus according to any one of claims 6 to 14, characterised in that it comprises means of introducing glass wool (11) into the furnace (1) via a lock which ensures that the furnace is maintained under negative pressure.